# EUROPEAN PATENT SPECIFICATION

(11) **EP 3 765 599 B1**
(45) Date of publication and mention of the grant of the patent: **14.12.2022**
(21) Application number: 19704845.7
(22) Date of filing: 20.02.2019
(51) Int. Cl.: C12N 5/077, C12N 5/071

(54) **PROCESS FOR PRODUCING CARDIAC ORGANOIDS**
VERFAHREN ZUR HERSTELLUNG VON HERZORGANOIDEN
PROCÉDÉ DE PRODUCTION D'ORGANOÏDES CARDIAQUES

(30) Priority: 13.03.2018 EP 18161619
(43) Date of publication of application: 20.01.2021
(73) Proprietor: Medizinische Hochschule Hannover, 30625 Hannover (DE)
(72) Inventor: ZWEIGERDT, Robert, 30625 Hannover (DE); DRAKHLIS, Lika, 30625 Hannover (DE)
(74) Representative: Taruttis, Stefan Georg
(86) International application number: PCT/EP2019/054225
(87) International publication number: WO 2019/174879

(56) References cited:
- WO-A1-2018/035574
- KR-A- 20170 141 995
- US-A1- 2016 215 264
- US-A1- 2016 271 183
- US-A1- 2017 198 256
- RICHARD J. MILLS ET AL: "Functional screening in human cardiac organoids reveals a metabolic mechanism for cardiomyocyte cell cycle arrest", PROCEEDINGS OF THE NATIONAL ACADEMY OF SCIENCES, vol. 114, no. 40, 15 September 2017 (2017-09-15), pages E8372-E8381, XP055488133, US ISSN: 0027-8424, DOI: 10.1073/pnas.1707316114
- RICHARDS DYLAN J ET AL: "Inspiration from heart development: Biomimetic development of functional human cardiac organoids", BIOMATERIALS, ELSEVIER SCIENCE PUBLISHERS BV., BARKING, GB, vol. 142, 12 July 2017 (2017-07-12), pages 112-123, XP085147126, ISSN: 0142-9612, DOI: 10.1016/J.BIOMATERIALS.2017.07.021 cited in the application
- PLANSKY HOANG ET AL: "Generation of spatial-patterned early-developing cardiac organoids using human pluripotent stem cells", NATURE PROTOCOLS, vol. 13, no. 4, 15 March 2018 (2018-03-15) , pages 723-737, XP055488131, GB ISSN: 1754-2189, DOI: 10.1038/nprot.2018.006

## Description

The present invention relates to an in vitro process for producing cardiac organoids using pluripotent stem cells (PSC), preferably human PSC, to the cardiac organoids obtainable by the process, and to a process for analysing the activity of a test compound on cardiac cells by exposing the PSC during the process for their production to the test compound and/or exposing at least one cardiac organoid obtainable by the process for their production to the test compound, and analysing the organoid, e.g. in comparison to PSC and/or a cardiac organoid treated in parallel but in the absence of the test compound.

The processes and the cardiac organoid have the advantage of being available on the basis of cultivated pluripotent stem cells, and have the advantage of providing adjoining layers comprising or consisting of different cardiac cells, optionally including non-cardiac cells, which layers are grown by a purely in vitro process, and to provide an in vitro model of adjoining cardiac tissue layers, optionally including non-cardiac cells. These tissue layers have been found to be well-structured and to contain cell types, which layers and cell types are typical of the early embryonic heart. The cardiac organoids can be kept in cell culture medium in static or agitated, e.g. shaken, culture vessels, e.g. without directional fluid flow, without blood flow, and freely suspended, i.e. without mechanical fixation.

### State of the art

It is known to cultivate cardiac cells in order to obtain layers of these cells which adhere to a cultivation vessel surface.

The trademark Matrigel (Corning Life Sciences and BD Bioscience) is known to designate a gelatinous protein mixture secreted by Engelbreth-Holm-Swarm (EHS) mouse sarcoma cells.

Richards et al., Biomaterials 112-123 (2017) describe the fabrication of cardiac organoids by depositing cardiomyocytes, cardiac ventricular fibroblast cells, umbilical vein endothelial cells (HUVEC), human adipose-derived microvascular endothelial cells (HAMEC), and human adipose-derived stem cells (hADSC) in agarose moulds to first generate spherical microtissues which are then assembled to spheroids.

Elliott et al. (2011) "NKX2-5(eGFP/w) hESCs for isolation of human cardiac progenitors and cardiomyocytes", Nature methods 8 (12), S. 1037-1040. DOI: 10.1038/nmeth.1740 describe the generation of hESC3 NKX2.5-eGFP cells.

US 2017/0198256 A1 describes that hiPSC were cultivated and harvested as single cells or cell clumps, which were combined with a liquid, cross-linkable PEG fibrinogen precursor solution to generate a statistical distribution of single hiPSC or clumps of hiPSC in the cross-linkable solution, which was cross-linked in forms to give cylinders of 6 mm width and 200 µm thickness. After cultivating these cylinders for 3 days, medium was changed to induce differentiation of the hiPSC, generating cardiac myocytes.

US 2016/0271183 A1 describes the differentiation of human embryonic stem cells in suspension in medium containing Matrigel with BMP4, Rho kinase inhibitor, activin-A and IWR-1 to cardiomyocytes.

WO 2018/035574 A1 and Mills et al., PNAS E8372-E8381 (2017) describe a process for mixing cardiomyocytes in Matrigel. The cells that are embedded are cardiomyocytes (p. 28, lines 1-33), and the mixture of cardiomyocytes and Matrigel is pipetted into the culture vessel that has two spaced-apart vertical poles. The resulting product is flat and elongate with spaced-apart holes corresponding to sites embracing the poles.

Pashmforoush et al., Nkx2-5 Pathways and Congenital Heart Disease: Loss of Ventricular Myocyte Lineage Specification Leads to Progressive Cardiomyopathy and Complete Heart Block, Cell 2004, 117, 373-386, describe the in vivo structural defects for genetic defects of NKX2.5.

Ng ES, Azzola L, Bruveris FF, Calvanese V, Phipson B, Vlahos K, et al., Differentiation of human embryonic stem cells to HOXA+ hemogenic vasculature that resembles the aorta-gonad-mesonephros. Nature Biotechnology 2016, 34(11), describe the hematopoietic cell-specific gene RUNX1C (Runt-related transcription factor 1c).

Guibentif C, Rönn RE, Boiers C, Lang S, Saxena S, Soneji S, et al. Single-Cell Analysis Identifies Distinct Stages of Human Endothelial-to-Hematopoietic Transition. Cell Reports 2017, 19, 10-19, describe the hematopoietic cell-specific gene WAS of the Wiskott-Aldrich syndrome.

Kasahara et al., "Progressive atrioventricular conduction defects and heart failure in mice expressing a mutant Csx/Nkx2.5 homeoprotein", 2001; J. Clin. Invest. 108:189-201, describe that mouse embryos and neonatal mice with a ventricle-restricted NKX2.5-KO contain cardiomyocytes with well-organized sarcomeres similar to wildtype mouse cardiomyocytes.

Davis et al., "Targeting a GFP reporter gene to the MIXL1 locus of human embryonic stem cells identifies human primitive streak-like cells and enables isolation of primitive hematopoietic precursors", Blood 2008, 111:1876-1884 describes the HES3 MIXL1-GFP reporter cell line.

Anderson DJ et al. "NKX2-5 regulates human cardiomyogenesis via a HEY2 dependent transcriptional network", Nature Communications 2018, 9: 1373 describe generation of the human embryonic stem cell line HES3 NKX2.5^{eGFP/eGFP}.

### Object of the invention

It is an object of the invention to provide an alternative process for producing cardiac tissue in an in vitro process, which cardiac tissue shall form at least two layers comprising or consisting of different cells, which can be cardiac cells, optionally in combination with other cell types.

### Description of the invention

The invention achieves the object by the features of the claims, especially by an in vitro process for producing a cardiac organoid and by the cardiac organoid obtainable by the process, the process comprising or consisting of the following order of steps of
a) providing cultivated pluripotent stem cells (PSC), preferably only PSC, wherein PSC preferably are human PSC, e.g. in a cell number of at least 50 or at least 100 or at least 1000 cells, e.g. up to 200,000 or up to 100,000 cells, e.g. 4,000 to 10,000 cells, preferably 5000 cells, in a suspension in a first culture medium, in a first vessel,
b) centrifuging the PSC in the first vessel having a U-shaped bottom to localize the PSC at the bottom of the first vessel,
c) incubating the PSC localized at the bottom of the first vessel under the first medium under cell culture conditions to form one aggregate of PSC,
d) optionally removing the first medium from the aggregate of PSC localized at the bottom of the first vessel,
e) embedding the aggregate of PSC in hydrogel, preferably by positioning a volume of hydrogel in a second vessel and transferring the aggregate of PSC localized at the bottom of the first vessel into the volume of hydrogel,
f) incubating the aggregate of PSC embedded within the hydrogel for solidifying the hydrogel, e.g. for at least 10 min, e.g. für 10 to 90 min or to 45 min, or for 45 to 90 min
g) covering the aggregate of cells embedded in the solidified hydrogel with a second cell culture medium and incubating under cell culture conditions, preferably for 1 to 3 d, e.g. for 36 to 60 h,
h) removing the second medium from the cells and adding a third cell culture medium containing a first differentiation factor having activity to induce the WNT pathway and incubating under cell culture conditions for at least 6 h, e.g. for up to 3 d, preferably for 12 to 48 h, e.g. for 24h,
i) removing the third medium from the cells and adding a fourth cell culture medium not containing the first differentiation factor or containing an agent neutralizing the activity of the first differentiation factor, and incubating under cell culture conditions for at least 6 h, e.g. up to 3 d, preferably for 12 to 48 h, e.g. for 24 h,
   a) removing the medium from the cells and adding a fifth cell culture medium containing a second differentiation factor having activity to inhibit the WNT pathway and incubating under cell culture conditions for at least 1 d or at least 2 d, preferably for 46 to 50 h, e.g. for 48 h,
   b) removing the fifth medium from the cells and adding a sixth medium not containing insulin and not containing a differentiation factor having activity to induce or to inhibit the WNT pathway and incubating under cell culture conditions for at least 1 d or at least 2 d, preferably for 2 days,
   c) removing the sixth medium from the cells and adding a seventh cell culture medium containing insulin and incubating under cell culture conditions for at least 1 d or at least 2 d, preferably for 2 days,
   d) and subsequently change the seventh medium for fresh cell culture medium at least every 2 d.

As an alternative, PSC can be replaced by non-human omnipotent or totipotent stem cells. Accordingly, in the process the stem cells can optionally be at least pluripotent stem cells. Generally, the PSC are generated without use of a human embryo. Preferably, PSC are human PSC (hPCS), e.g. induced PSC (iPSC), e.g. generated from a mammalian cell sample, e.g. a blood or tissue sample, especially a human induced PSC (hiPSC), or an embryonic stem cell line (ESC), which preferably is non-human, or a human embryonic stem cell line (hESC). Generally, the PSC or ESC are not generated using a human embryo.

Cultivated pluripotent stem cells (PSC) preferably are characterized by presence of the markers Tra-1-60 and SSEA4.

Preferably, the cultivated pluripotent stem cells (PSC) provided in step a) are single cells in suspension.

The first vessel preferably has a low-attachment surface, preferably an ultra-low attachment surface which prevents attachment of the PSC to the vessel. The first vessel can e.g. be contained in a microtiter plate, e.g. in a 96-well plate. The second vessel preferably has a low-attachment surface, preferably an ultra-low attachment surface which prevents attachment of the PSC to the vessel. The second vessel may have a flat or U-shaped bottom. The first vessel and the second vessel can have the same shape and/or surface properties.

Generally, the U-shaped bottom of the first and second culture vessels can be a tapering concave bottom, e.g. round or stepped or having angled surfaces, e.g. having a concave pyramid shape or V-shape.

The first medium preferably is E8 medium, having the composition of DMEM/F 12, L-ascorbic acid-2-phosphate magnesium (64 mg/L), sodium selenium (14 µg/L), FGF2 (100 µg/L), insulin (19.4 mg/L), NaHCO₃ (543 mg/L) and transferrin (10.7 mg/L), TGFβ1 (2 µg/L) or NODAL (100 µg/L). Osmolarity of all media was adjusted to 340 mOsm at pH7.4, in order to provide for pluripotency of the ESC. Preferably, the first medium is supplemented with ROCK inhibitor, e.g. 10 µM ROCK inhibitor (Rho-associated-kinase inhibitor).

The volume of each medium can be in the range of 100 µL to 300 µL, especially 150 µL to 250 µL for every 5000 cells.

The centrifugation in step b), e.g. twice at 300 x g in a centrifuge temperature-controlled to 4°C, serves to localize the PSC on the bottom of the first vessel, e.g. to collect the PSC. Following the centrifugation, the first vessel is placed in an incubator essentially without moving the first medium in relation to the first vessel, in order to maintain the PSC localized close together. The first medium remains in the first vessel, also in step c).

The incubation of the PSC localized on the bottom of the first vessel under cell culture conditions and without agitation in step c). This incubation, e.g. for 24h, results in the PSC to form one aggregate of cells. In step c) the PSC localized at the bottom of the first vessel under the first medium under cell culture conditions can be incubated for 46 to 50 h.

Removing the first medium from the aggregate of cells in step d) serves to minimize or avoid the transfer of first medium into the hydrogel that is positioned in the U-shaped bottom of a second vessel in step e), when transferring the aggregate of cells into the hydrogel in step f). Transferring the aggregate of cells in step e) can e.g. be done using a pipette having a wide opening in order to avoid shearing the aggregate of cells.

The hydrogel is for cell culture, e.g. is permeable for dissolved oxygen and permeable for medium components and preferably permeable for metabolic products, e.g. permeable for diffusion. Preferably, the hydrogel is on the basis of an extracellular matrix of basal membranes of animal cells. The hydrogel can be on the basis of laminin and/or entactin and/or collagen and/or fibronectin and/or PEG, optionally on the basis of PEG (polyethylene glycol), e.g. in combination with fibronectin. The hydrogel preferably has a gelatinous structure. A preferred hydrogel is Matrigel.

The hydrogel, e.g. Matrigel, preferably has a protein concentration of 9.7 to 10.1 mg/mL. In step e), preferably a volume of 15 to 30 µL, preferably 20 µL Matrigel is positioned onto the U-shaped bottom of a second vessel and the aggregate of PSC is positioned into the Matrigel for embedding the aggregate of PSC within the Matrigel. The second vessel preferably has a low-attachment surface, preferably an ultra-low -attachment surface. The second vessel can have the same specifications as the first vessel. Preferably, the hydrogel is not cross-linkable, e.g. does not contain reactive groups that cross-link during the process, and accordingly, the process preferably is devoid of a step of cross-linking the hydrogel. Less preferred, the hydrogel may be cross-linkable, e.g. contain cross-linkable groups, and the process comprises a step of cross-linking the hydrogel.

The incubation of step f), e.g. for 45 to 60 min under cell culture conditions, serves to solidify the hydrogel prior to adding the second culture medium in step g).

In step g), the second medium can have the same composition as the first medium, e.g. medium E8. The volume of the second medium in step g) can be 80 to 150 µL, e.g. 100µL. The incubation of step g) is for at least 1 day or at least two days, preferably up to 3 days, preferably for 36 to 60 h.

Removal of the second medium and adding the third medium containing a first differentiation factor in step i) serves to activate the WNT pathway. The first differentiation factor having activity to induce the WNT pathway preferably is an inhibitor of GSK3beta (glycogen synthase kinase 3 beta), and preferably has no effect or cross-reactivity on CDKs (cyclin-dependent kinases). A preferred first differentiation factor is CHIR99021 (CHIR, 6-[[2-[[4-(2,4-dichlorophenyl)-5-(5-methyl-1H-imidazol-2-yl)-2 pyrimidinyl]amino]ethyl]amino]-3-pyridinecarbonitrile), e.g. at 7.5µM, e.g. using 200µL of the third medium per second vessel. The third medium preferably is RPMImedium containing B27 supplement without insulin and it additionally contains the first differentiation factor.

Preferably, after step h), the third medium containing the first differentiation factor is removed in step i), preferably at 24h after adding this third medium in step j), and a fourth cell culture medium is added, which does not contain a differentiation factor, e.g. no first differentiation factor, and incubating under cell culture conditions, e.g. for 1d. When the process includes step i), this is presently considered to reduce the WNT activation induced the first differentiation factor. The fourth medium preferably is free from insulin, e.g. RPMI medium containing B27 supplement but without insulin. Generally preferably, in the process, especially in step h), only a first differentiation factor having activity to activate the WNT pathway only is added, e.g. contained in a third cell culture medium, and the process, especially in step h), is devoid of adding a differentiation factor having activity to activate another differentiation pathway than the WNT pathway, e.g. devoid of adding BMP4, Rho kinase inhibitor, activin-A and IWR-1.

In step j), the medium is removed from the cell aggregate and a fifth cell culture medium is added, which contains a second differentiation factor which inhibits the WNT2 pathway, and preferably the fifth medium contains no insulin. The fifth medium can be RPMI medium containing B27 supplement but without insulin. The second differentiation factor preferably is an inhibitor of the WNT pathway activator Porcupine, e.g. IWP2 (Inhibitor of WNT Production-2, CAS No. 686770-61-6), e.g. at a concentration of 5µM, e.g. using 100µL to 300 µL, e.g. up to 200 µL medium, per second vessel.

In step 1), preferably after 2 d incubation of step k), the fifth medium is removed and a sixth medium is added to the agglomerate, which medium does not contain a first nor a second inhibitor. The sixth medium preferably contains no insulin. The sixth medium can e.g. be RPMI medium containing B27 supplement without insulin. In step k), the volume of the sixth medium can e.g. be 100 µL to 300 µL, e.g. 150 to 250 µL, preferably 200 µL per second vessel. RPMI medium is RPMI1640 (Inorganic Salts: Calcium nitrate ^{∗} 4H₂O (0.1 g/L), magnesium sulfate (0.04884 g/L), potassium chloride (0.4 g/L), sodium bicarbonate (2 g/L), sodium chloride (6 g/L), sodium phosphate dibasic (0.8 g/L); Amino Acids: L-alanyl-L-glutamine (0 g/L), L-arginine (0.2 g/L), L-asparagine (0.05 g/L), L-aspartic acid (0.02 g/L), L-cystine ^{∗} 2HCl (0.0652 g/L), L-glutamic acid (0.02 g/L), glycine (0.01 g/L), L-histidine (0.015 g/L), hydroxy-L-proline (0.02 g/L), L-isoleucine (0.05 g/L), L-leucine (0.05 g/L), L-lysine ^{∗} HCI (0.04), L-methionine (0.015 g/L), L-phenylalanine (0.015 g/L), L-proline (0.02 g/L), L-serine (0.03 g/L), L-threonine (0.02 g/L), L-tryptophan (0.005 g/L), L-tyrosine ^{∗} 2Na ^{∗} 2H₂O (0.02883 g/L), L-valine (0.02 g/L); Vitamins: D-biotin (0.0002 g/L), choline chloride (0.003 g/L), folic acid (0.001 g/L), myo-inositol (0.035 g/L), niacinamide (0.001 g/L), p-aminobenzoic acid (0.001 g/L), D-phantothenic acid (hemicalcium) (0.00025 g/L), pyridoxine ^{∗} HCl (0.001 g/L), riboflavin (0.0002 g/L), thiamine ^{∗} HCI (0.001 g/L), vitamin B12 (0.000005 g/L); D-glucose (2 g/L), glutathione (0.001 g/L), phenol red ^{∗} Na (0.0053 g/L); L-Glutamine (0.3 g/L), sodium bicarbonate (0 g/L)).

In step 1), the sixth medium is removed and replaced by a seventh medium which contains insulin, e.g. RPMI medium containing B27 supplement with insulin added.

In step m) the seventh medium of step 1) is replaced by fresh cell culture medium, preferably fresh seventh medium, which preferably contains added insulin.

Preferably, the incubation in steps c) to m), preferably in steps c) to h), are under static conditions, e.g. without shaking or moving of the vessel. Optionally, the incubation starting from step m) are with mild agitation, e.g. under orbital shaking, preferably in a larger volume of medium and in a vessel for cell culture with orbital shaking.

The process for producing the cardiac organoids has the advantage that only one type of cells, namely PSC, e.g. iPSC or ESC, can be used in the process and that during the process the PSC, e.g. iPSC or ESC, differentiate and self-organize into a three-dimensional structure which comprises adjoining layers comprising or consisting of different cardiac cell types, e.g. without mechanically manipulating cells into a specific layered structure, and without initially providing different cardiac cell types.

Herein, the gelatinous protein mixture secreted by Engelbreth-Holm-Swarm (EHS) mouse sarcoma cells is generally referred to as Matrigel.

Generally, all media can contain anti-bacterial agents, e.g. penicillin and/or streptomycin for the prevention of bacterial contaminations.

For analysing the effect of a test compound, the test compound can be added in any of the steps of the process. For analysing the effect of a test compound, the test compound can be added to the medium in at least one of steps e) to m, e.g. in at least one of steps i) to m), preferably after a total of 10 to 13 days after step i).

The process for producing the cardiac organoids has the advantage of reproducibly generating cardiac organoids of one structure, e.g. having an outer diameter of 0.5 to 5 mm or up to 3 or up to 2.5 mm, e.g. of 1.2 to 3 mm or up to 2.5 mm. The structure of the cardiac organoids contains or consists of a first layer, comprising or essentially consisting of endothelial cells and foregut endoderm cells, which first layer forms an inner part and has cavities, which preferably comprise hemogenic endothelium and/or foregut endoderm cells, a second layer directly adjacent the first layer and surrounding only a portion of the first layer, the second layer forming dense myocardium, optionally also endocardium, comprising or essentially consisting of NKX2.5-positive cardiomyocytes, and opposite the first layer a third layer directly adjacent to the second layer only and surrounding only a portion of the second layer, which third layer forms epicardium and comprises or essentially consists of epicardial cells and cardiomyocytes, has blood vessel-like structures and forms a loose tissue from epicardial cells and cardiomyocytes, wherein the third layer can be covered by a directly adjacent fibroblast layer. Therein, preferably the first layer completely surrounds the cavity or cavities, i.e. the first layer is circumferentially closed, and the second layer surrounds the first layer only partially, leaving a portion of the first layer non-covered from another layer. The third layer surrounds the second layer only partially, e.g. the third layer covers the second layer up to a ring-shaped section of the second layer, which ring-shaped section of the second layer is non-covered, which ring-shaped section is adjacent to the non-covered portion of the first layer. Hemogenic endothelium can e.g. be localized in the inner part of the cardiac organoids, especially at least a portion of inner cavities, e.g. in only a portion of inner cavities with another portion of cavities being devoid of hemogenic endothelium, which can be foregut endoderm cells. The portion of cavities having hemogenic endothelium can have a layer of hemogenic endothelium lining the cavities, with the inner part being formed of foregut endoderm cells. The portion of cavities having no hemogenic endothelium are only formed of foregut endoderm cells. The inner part of the cardiac organoids can be formed of foregut endoderm cells, wherein separate cavities can be formed of foregut endoderm cells, be lined with hemogenic endothelium, and/or contain blood vessels.

The structure of the cardiac organoids therefore comprises or consists of, e.g. in one perspective, at least one non-covered portion of the first layer surrounded by a ring-shaped section of the second layer, wherein the second layer is covered by the adjacent third layer opposite the first layer, and the ring-shaped section of the second layer is not covered by the third layer. The structure of the cardiac organoids preferably has at least one surface area which is formed by a non-covered portion of the first layer, by a ring-shaped section of the second layer directly adjacent the first non-covered portion of the first layer, and directly adjacent to the ring-shaped section of the second layer, the third layer which covers and surrounds the second layer, the third layer bordering on the ring-shaped section of the second layer, and preferably a fourth layer, e.g. of loose cardiac fibroblasts, only adjacent the third layer. Optionally, the first layer in its part directly under its non-covered portion contains less or no cavities than its portions which are covered by the second layer. In the structure of the cardiac organoids, the at least one surface area can be formed by the ring-shaped section of the second layer, which encloses the non-covered portion of the first layer and, opposite the non-covered portion of the first layer, by the third layer surrounding the ring-shaped section of the second layer, wherein the third layer preferably covers the second layer completely and borders on the ring-shaped section of the second layer.

As the process for producing the cardiac organoids reproducibly generates the cardiac organoids having this structure, the effect of a test compound added during the process or to cardiac organoids having a pre-determined size, can be analysed, e.g. by determining structural differences to cardiac organoids produced without the addition of the test compound.

The structure of the cardiac organoids has the advantage of allowing direct observation of all the layers from the perspective directed onto the non-covered portion of the first layer and/or onto the ring-shaped section of the second layer. Accordingly, the invention provides the cardiac organoids having the specific structure, which cardiac organoids are available by the process. Further, the invention provides analytical processes in which a compound to be tested is added to at least one of the media in at least one of the steps of the process of producing the cardiac organoid, or in which a compound to be tested is added to a cardiac organoid produced by the process, e.g. the cardiac organoid having a structure described herein, followed by analysis of the cardiac organoid.

Optionally, a section of the cardiac organoids, e.g. at least one of its layers, can be provided for use as a tissue implant for implantation into a patient, e.g. provided as a tissue implant, e.g. as a replacement or filler for a defect tissue.

Optionally, the pluripotent stem cells originate from a patient, e.g. from a tissue sample taken from a patient. The patient can be diagnosed to have a disease or can be suspected of having a disease, and the process for producing the cardiac organoids can be used to analyse the effect of a test compound onto the cardiac organoids, e.g. during or following their development to a pre-determined size, wherein the test compound can be a pharmaceutical compound for use in the treatment of the disease. Such a process can be used to analyse the effect of the pharmaceutical compound prior to administration of the compound to the patient. Alternatively or additionally, cardiac organoids produced according to the process of the invention from pluripotent stem cells that originate from a patient can be used to produce a cardiac organoid for use of at least one of its layers as a replacement for a defect tissue in the patient.

Optionally, the PSC may have a genetic aberration, e.g. a genetic defect selected from an aberrant, e.g. dysfunctional gene, which is present heterozygously or homozygously. The genetic aberration can e.g. be a dysfunctional gene selected from the genes of the group NKX2.5, a dysfunction of which at least homozygously results in congenital heart disease with structural defects including muscle overgrowth and conduction defects in the heart, a dysfunction of a factor such as GATA4 or TBX5, which lead to congenital heart defects. Analyses could demonstrate that genetic aberrations of the PSC used in the process result in structural aberrations of cardiac organoids in comparison to cardiac organoids produced from PSC without genetic aberrations, i.e. from genetically wild-type, or healthy, PSC.

The PSC can be a cell line, e.g. the hESC HES3, which is cited for reference only.

The invention is now described in greater detail by way of examples with reference to the figures, which show in
- Fig. 1 a schematic representation of a preferred embodiment of the process,
- Fig. 2 FACS results for an exemplary PSC cell line,
- Fig. 3 fluorescence microscopic picture of a cardiac organoid obtained on day 14 of the process (d10),
- Fig. 4 fluorescence microscopic pictures of different cardiac organoids on day 14 of the process,
- Fig. 5A a fluorescence microscopic picture of a cardiac organoid using immunostaining of Calponin-positive myofibroblasts at the perimeter of the outer layer of cardiac organoids,
- Fig. 5B a cardiac organoid in a whole-mount immunofluorescence staining using an anti-Vimentin antibody,
- Fig. 6 expression of WT1 in the outer layer only of cardiac organoids, 6A in immuno-staining, and 6B expression of a reporter gene,
- Fig. 7 a fluorescence microscopic picture of a cardiac organoid using immunostaining of cTnT in a paraffin section of a cardiac organoid,
- Fig. 8 cardiac organoids with staining for endothelial cells, in 8A a fluorescent micrograph for NKX2.5-eGFP expression with DAPI staining, in 8B a fluorescent micrograph with staining for CD31 and DAPI staining, in 8C a fluorescent micrograph with NKX2.5-eGFP expression, staining for CD31 and with DAPI staining,
- Fig. 9 a micrograph of a cardiac organoid with immunostaining for SOX17,
- Fig. 10 a micrograph of a paraffin section of a cardiac organoid with DAPI staining,
- Fig. 11A and 11B light micrographs and Fig. 11C an electron micrograph in a section of a cardiac organoid in the region of a large cavity,
- Fig. 12 an electron micrograph of a section of a large cavity of a cardiac organoid,
- Fig. 13 a micrograph of a cardiac organoid after long-term culture stained with Dil-Ac-LDL,
- Fig. 14A-C a schematic representation of the structure of cardiac organoids produced by the process of the invention,
- Fig. 14D microscopic pictures taken from different angles of one cardiac organoid,
- Fig. 15 micrographs of cardiac organoids treated with Thalidomide and untreated cardiac organoids,
- Fig. 16 graphically shows quantitative differences in the structure of cardiac organoids treated with Thalidomide and without test compound added,
- Fig. 17 fluorescence microscopic pictures of cardiac organoids produced from a genetically aberrant PSC or from a Control,
- Fig. 18 measurement results for total area occupied by cardiac organoids produced from a genetically aberrant PSC or from a Control,
- Fig. 19 and Fig. 20 fluorescence microscopic pictures of one optical analysis of the compactness of the ring tissue of cardiac organoids produced from a genetically aberrant PSC or from a Control,
- Fig. 21 the measurement result of an optical analysis of the compactness of the ring tissue of organoids produced from a genetically aberrant PSC or from a Control as shown in Fig. 19 and 20,
- Fig. 22 a fluorescence microscopic picture of a cardiac organoid expressing GFP under the control of the RUNX1C promoter,
- Fig. 23 a fluorescence microscopic picture of a cardiac organoid expressing GFP under the control of the WAS promoter,
- Fig. 24a and Fig. 24b an enlarged section of a fluorescence microscopic picture of a cardiac organoid with immune staining for endothelial cells,
- Fig. 25 shows transmission electron microscopy pictures of sarcomeres in cardiomyocytes in NKX2.5-KO and control organoids,
- Fig. 26 paraffin sections of cardiac organoids produced from NKX2.5-KO and wild-type (control) PSC, stained with an anti-Ki67 antibody, and in
- Fig. 27 microscopic pictures on day 1 (d1) to day 4 (d4) of differentiation of cardiac organoids produced from the HES MIXL1-GFP reporter cell line.

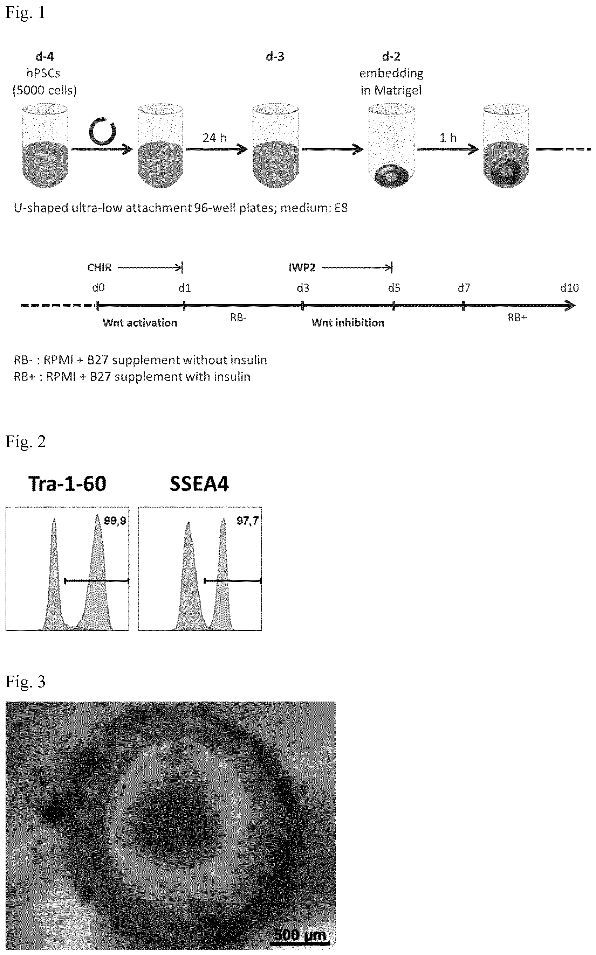

Fig. 1 shows a schematic overview of the process, wherein the sequence of steps is counted from the start of step h) as day 0. Therein, step a) is performed at day -4 (d-4) by centrifuging approx. 5000 human PSC (hPSCs) in the wells of a 96-well microtiter ultra-low attachment plate with U-shaped bottom as the first vessel to generate the aggregate of PSC at the U-shaped bottom of step b). The aggregate of PSC are incubated in the medium in step c) for the preferred duration of 24 h, ending on day -3 (d-3). The result of steps d) and e), which is the cell aggregate obtained on d-3 embedded in hydrogel in the bottom of a second vessel, is shown on day -2 (d-2) as the result of step e). The cell aggregate embedded in hydrogel is incubated for 1 h under cell culture conditions in step f), without medium contained in the second vessel. In step g) the second medium is added and incubation is for 2 d until on day 0 (d0) in step h) the second medium is replaced by the third medium containing the first differentiation factor to start the WNT pathway, with incubation in the third medium for 1 day (until day 1 (d1)). The third medium was 200 µL with 7.5 µM CHIR99021 per second vessel.

The step i) of replacing the third medium for a fourth medium that is free of a differentiation factor, is made on day 1 and incubation in the fourth medium is for 2 days until day 3 (d3). In the alternative, step i) can be performed with an incubation of 1 to 2 days, preferably 24 h under cell culture conditions, and the third medium is subsequently directly removed and exchanged for the fifth medium containing an inhibitor of the WNT pathway, IWP2 at 5µM, 200 µL medium per second vessel in step j). Incubation was for 2 days until day 5 (d5), when in step k) the fifth medium was removed and replaced by a sixth medium which is free from a differentiation factor and preferably contains no insulin, and incubation is for 2 days until day 7 (d7). The sixth medium is replaced by a seventh medium, which is generally free from differentiation factors and preferably contains insulin, e.g. B27 supplement, followed by incubation for at least 1 day, e.g. for up to 3 days, until day 10 (d10) in step 1). Following step 1), the sixth medium is exchanged for fresh medium, preferably containing insulin, in order to maintain viability, e.g. every day to every 2 days in step m).

In Fig. 1, RB- is RPMI medium containing B27 supplement without insulin, RB+ is RPMI medium containing B27 supplement, which contains insulin. B27 supplement, e.g. available from ThermoFisher Scientific, contains biotin, DL-alpha-tocopherol acetate, DL-alpha-tocopherol, vitamin A (acetat), BSA, fatty acid free Fraction V, catalase, human recombinant insulin, human transferrin, superoxide dismutase, corticosterone, D-galactose, ethanolamine HCl, glutathione (reduced), L-carnitine HCl, linoleic acid, linolenic acid, progesterone, putrescine 2HCl, sodium selenite, T3 (triiodo-l-thyronine). An insulin-free variant of B27 supplement is commercially available.

Reference Example 1: In vitro production of cardiac organoids only from PSC The process was performed for steps a) to n) as described with reference to Fig. 1. Incubation under cell culture conditions was in a 5% CO₂ atmosphere at 37°C, using ultra-low attachment 96 well microtiter plates, Nunclon Sphera 96U-well plate available from Thermo Fisher Scientific, as the first vessels. The PSC were the human embryonic stem cell line hESC3 NKX2.5-eGFP. These exemplary PSC were genetically manipulated to express enhanced green fluorescent protein (eGFP) as a reporter under the control of the early cardiomyocyte-specific marker NKX2.5 to indicate early cardiomyocyte differentiation. These PSC were cultivated on cell culture flasks (preferably Geltrex-coated) in E8 medium and detached from the flasks to provide the PSC in suspension. In step a), 5000 PSC suspended in 100 µL of the first medium, which was E8 medium (DMEM/F12, L-ascorbic acid-2-phosphate magnesium (64 mg/L), sodium selenium (14 µg/L), FGF2 (100 µg/L), insulin (19.4 mg/L), NaHCO₃ (543 mg/L) and transferrin (10.7 mg/L), TGFβ1(2 µg/L) or NODAL (100 µg/L), osmolarity adjusted to 340 mOsm at pH7.4) supplemented with 10µM ROCK inhibitor (Y-27632, catalogue No. 72302, Stemcell Technologies) ), were carefully dispensed into wells of the microtiter plate. Centrifugation of step b) was twice for 3 min at 300 x g with the centrifuge cooled to 4°C. The centrifuged first vessels each contained one agglomerate on the U-shaped bottom and were carefully placed into a cell culture incubator in order to avoid disturbance of the cells localized at the U-shaped bottom. Incubation of step c) without agitation was for 24 h, resulting in the formation of an aggregate of the cells. For embedding in Matrigel (thawed on ice, 9.7 to 10.1 mg/mL protein content) as the exemplary hydrogel, 20 µL Matrigel were positioned in each well of a microtiter plate, the wells of which forming second vessels, which plate was of the same ultra-low attachment quality as the first. As preferred, the medium was removed from the cell aggregates contained in the first vessels, and the cell aggregates were singly and carefully transferred into the Matrigel droplets contained in the second vessels. It was found that for the transfer of a cell aggregate, a pipette set to approx. 3µL and equipped with a cut tip having a large opening could be used. The second vessel, i.e. the second microtiter plate, was re-positioned into the incubator for 45 to 60 min, which allowed for a solidification of the Matrigel. Then, 100 µL E8 medium but without ROCK inhibitor was dispensed into each second vessel and the second vessel was incubated for 2 days without agitation according to step h). After the incubation of step h), on day 0, differentiation was started by completely removing the E8 medium and adding 200 µL RPMI medium supplemented with B27 supplement without insulin and containing 7.5 µM CHIR99021 into each second vessel, and incubated for exactly 24h according to step h). Then, this third medium was exchanged for a fourth medium consisting of RPMI medium with B27 supplement without insulin, and incubation was for 2d. Then, the fourth medium was removed and exchanged for 200 µL RPMI medium with B27 supplement without insulin and containing 5µM IWP2 as the second differentiation factor in each second vessel according to step k), with incubation for 48 h. Subsequently, the medium was exchanged every 2 days for RPMI medium with B27 supplement containing insulin.

Fig. 2 shows the result of a FACS analysis of the suspended PSC cells with staining for the pluripotency markers Tra-1-60 (99.9% of cells positive) and SSEA4 (97.7% of cells positive), confirming pluripotency of the cells at the beginning of the process.

Fig. 3 shows a fluorescence microscopic picture of a typical cardiac organoid generated from the reporter hESC line HES3 NKX2.5^{eGFP}. The cardiac organoid shows an inner part forming an inner layer 1, which is comprised of NKX2.5-eGFP-negative cells. The inner layer is surrounded by a dense ring of NKX2.5-eGFP-positive cardiomyocytes, which can also be described as a myocardial ring. The dense ring is surrounded by a ring-like structure of an outer layer, which contains both NKX2.5-eGFP-positive and NKX2.5-eGFP-negative cells. The outer layer seems to be loose tissue.

It was generally observed that the cardiac organoids start to regularly contract after approx. 11 days of the process (day 7).

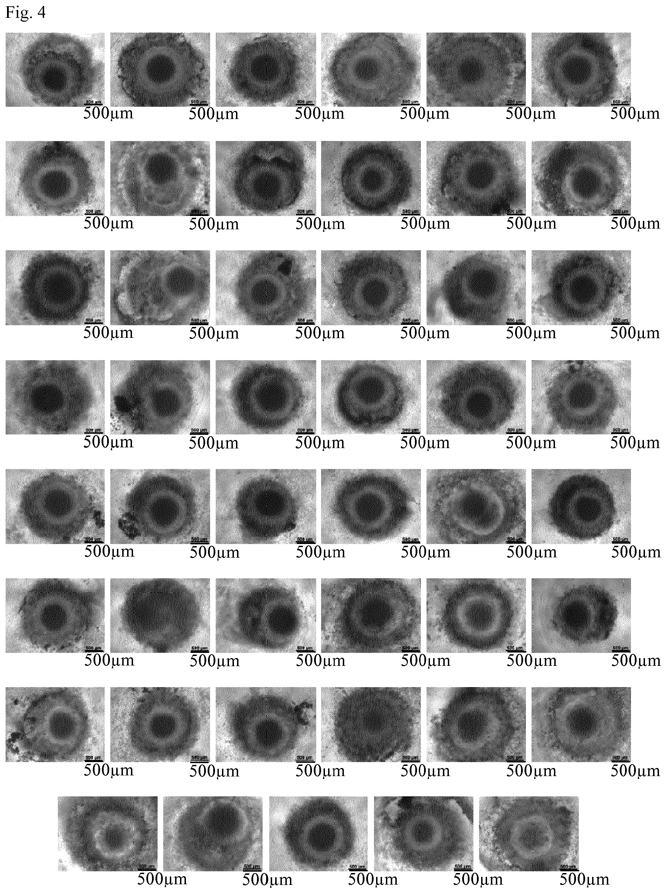

Fig. 4 depicts fluorescence microscopic pictures of different cardiac organoids on day 14 (d10) of the process, showing very similar structures and NKX2.5-eGFP expression for the organoids produced in parallel in one batch. This results shows that the process reproducibly generates the cardiac organoids having a similar structure.

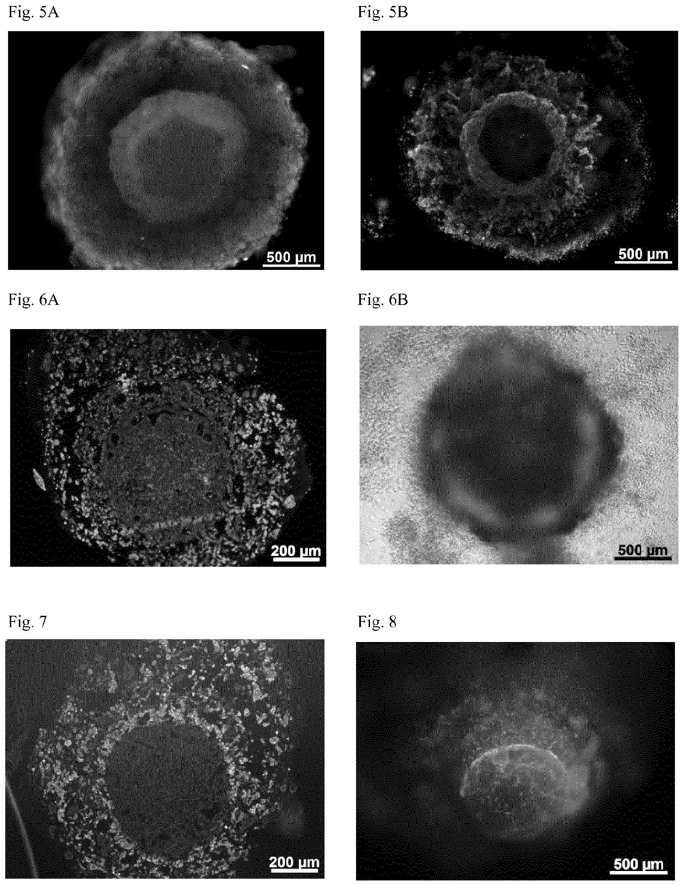

Fig. 5A depicts a cardiac organoid in a whole-mount immunofluorescence stain using a Cy5-labelled anti-Calponin antibody (Calponin 1 antibody (CALP): sc-58707 von Santa Cruz Biotechnology), DAPI staining and fluorescence of NKX2.5-eGFP. This result shows that the cardiac organoids of the invention contain Calponin-positive myofibroblasts at the perimeter of the outer layer.

Fig. 5B shows a cardiac organoid in a whole-mount immunofluorescence staining using an anti-Vimentin antibody (obtained from abcam; ab92547; secondary antibody: Alexa488-AffiniPure Donkey Anti-Rabbit IgG (H+L), obtained from Jackson ImmunoResearch Laboratories) and an anti-cTnT antibody (Cardiac Troponin T Antibody (13-11) obtained from Invitrogen; secondary antibody: Cy3-AffiniPure Donkey Anti-Mouse IgG (H+L) obtained from Jackson ImmunoResearch Laboratories). The staining shows that cardiac organoids contain Vimentin-positive fibroblasts, which are an example of stromal cells, at the perimeter of the outer layer.

Fig. 6 depicts micrographs of a cardiac organoid with WT1 immunostaining using an Alexa488-labelled anti-WTl antibody with DAPI staining in a paraffin section in 6A, and in 6B an immunofluorescence micrograph of a cardiac organoid expressing eGFP under the control of the WT1 promoter (HES3 WT1^{eGFP} cells). These results show that epicardial cells in the cardiac organoids are exclusively found in the out layer and not in other parts of the organoids.

Fig. 7 shows a paraffin section of a cardiac organoid with immuno-staining of the myocardial marker troponin T (cTnT) using a Cy3-labelled anti-cTnT antibody (Cardiac Troponin T Antibody (13-11) obtained from Invitrogen), with DAPI staining. This result shows that the cardiac organoids contain a dense ring of cTnT-positive cells between the outer layer and the inner part. This dense ring can consist of cTnT-positive cells, which are myocardial cells. Further, cTnT-positive cells are found in the outer layer.

Fig. 8 shows a whole-mount fluorescence micrograph of a cardiac organoid expressing NKX2.5-eGFP with DAPI staining and immunofluorescence staining for CD31 using a Cy3-labelled anti-CD31 antibody (CD31, Endothelial Cell (Concentrate) Clone JC70A obtained from Agilent, Dako). This shows that the cardiac organoids contain endothelial cells in their inner part.

Further, Figure 8 shows a line between the myocardial ring and the inner part, which could show a dense layer of endothelial cells, and which could indicate endocardium. The network structures of endothelial cells within the cardiac organoids can be regarded to form a primitive blood vessel network. Therefore generally, the cardiac organoids are especially suitable for analysing the effect of a test compound on the cardiac organoids, e.g. adding a test compound during the process in order to analyse the influence of the test compound on the development of the cardiac organoids, e.g. in comparison to a cardiac organoid produced in parallel without the addition of the test compound. The test compound can also be tested in respect of its influence on the development of the primitive blood vessel network of the cardiac organoids, e.g. for detecting and analysing an inhibitory or a stimulating effect of the test compound on blood vessel development.

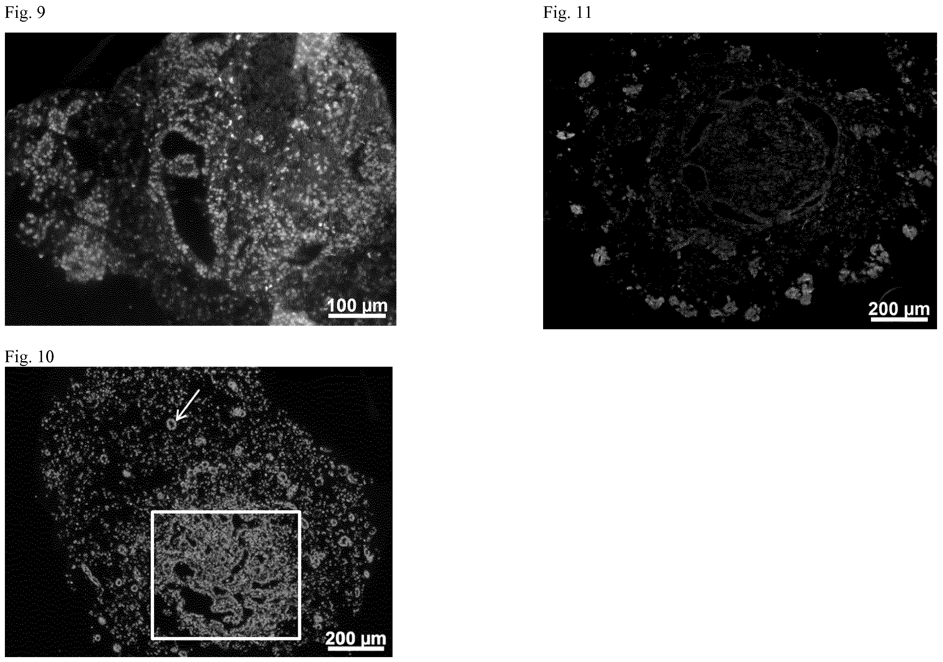

Fig. 9 shows a micrograph of a cardiac organoid in a section with immunostaining for SOX17 using a labelled anti-SOX17 antibody (Human SOX17 Antibody (AF1924) obtained from R&D Systems). This section shows that endothelial cells of the endothelial network inside the cardiac organoids are SOX17-positive. Noticeably, SOX17-positive endothelial cells are found in the aorta-gonad-mesonephros region of the developing embryo. SOX17 does not only mark hemogenic endothelium but also foregut endoderm. In the human embryo, the heart develops in close proximity to the foregut endoderm, which is known to give rise to the anlagen of the lung, liver, thymus, thyroid, esophagus and stomach. Accordingly, in the cardiac organoids, the "cardiac" part comprising myocardium-, epicardium- and putatively endocardium-anlagen develops in close proximity to the SOX17 positive inner part, suggesting that the cardiac organoids in their inner part comprise foregut endoderm anlagen. Fig. 10 depicts a micrograph of a paraffin section of a cardiac organoid with DAPI staining. This shows that the organoids contain large cavities in the center of their inner part, highlighted by the drawn box, and small, vessel-like structures in the outer loose tissue, indicated by the arrow.

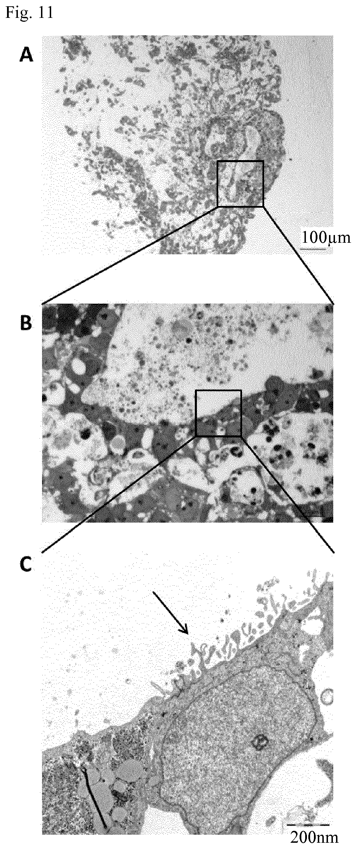

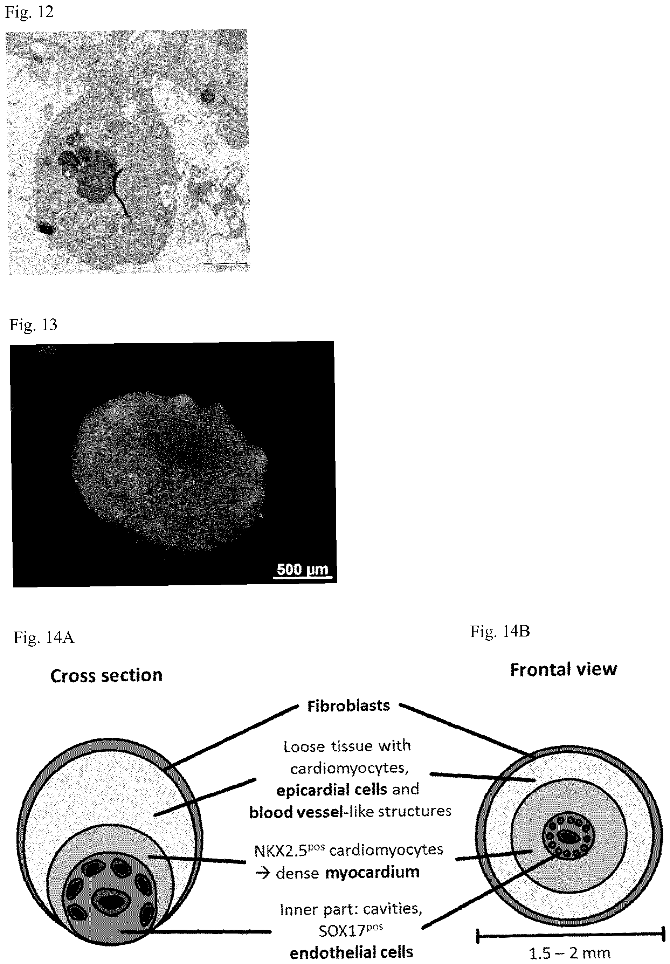

Fig. 11A and the enlargement in Fig. 11B show semi-thin sections of cardiac organoids embedded in epoxy resin, in staining with toluidine blue in light microscopy, and in Fig. 12C enlarged by electron microscopy. These pictures show that the large cavities of the cardiac organoids in the inner part are lined with epithelium having pseudopodia (indicated by the arrow in Fig. 11C).

The electron micrograph of Fig. 12 shows a cell that was found within a large cavity of a cardiac organoid. This cell has a morphology suggesting a macrophage phenotype. This finding supports that the inner part of the cardiac organoids contains hemogenic endothelium.

### Reference Example 2: Long-term culture of cardiac organoid

After 14 days of the process, i.e. on day 10, cardiac organoids produced according to Example 1 were transferred to wells of a 12-well plate containing PBS (phosphate-buffered saline, pH 7.4), the PBS was removed by suction and the Matrigel was dissolved by adding Cell Recovery Solution, obtained from Corning, according to the manufacturer's instructions. Then, the organoid was gently washed with PBS, PBS was removed, and 2 mL RPMI medium with B27 supplement and containing insulin was added into each well, then the plate was incubated further, with exchange of the medium for fresh medium every 3 to 4 days. The organoid shown in Fig. 13 was kept in this culture until day 146 (i.e. 146 days starting from step i), then stained with Dil-AC-LDL (obtained from Thermo Fisher Scientific). Staining procedure: DiI-Ac-LDL was added directly to the medium (RB+) to a final concentration of 5 µg/mL. Organoids were stained in a 12-well plate in a volume of 2 mL. Incubation with the DiI-Ac-LDL: 4 h in the incubator. Afterwards, the medium with the DiI-Ac-LDL was removed and fresh medium (RB+) without the dye was added. For imaging, medium was replaced by PBS. The fluorescence micrograph of Fig. 13 shows that the organoid still contains endothelial cells and expresses NKX2.5-eGFP. The cardiac organoid showed contractions until the end of the culture.

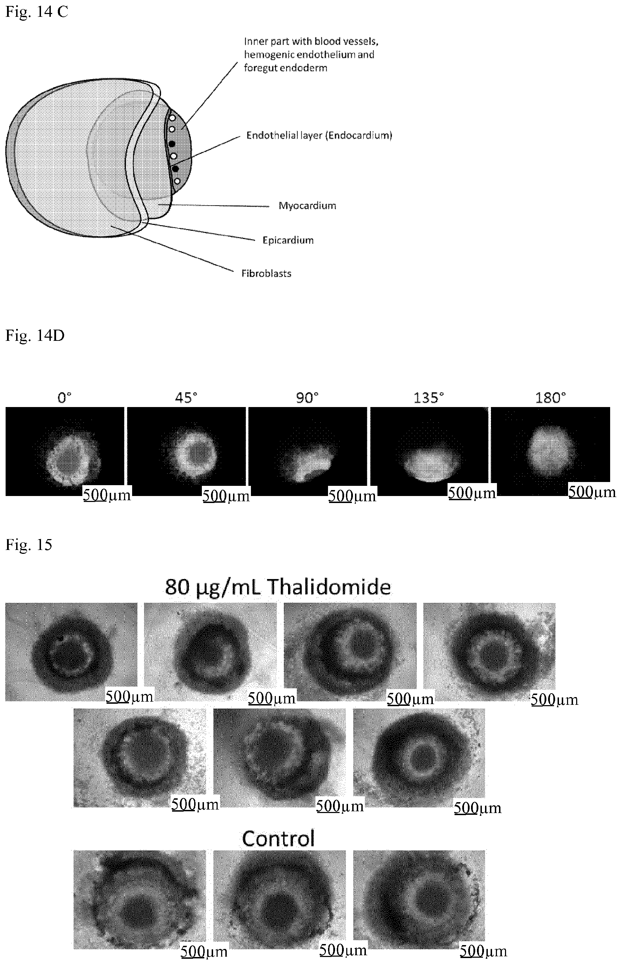

Figures 14A-14C show a schematic representation of the structure of the cardiac organoids which could be derived from the analytical results. The cardiac organoids produced by the process of the invention contain or consist of a first layer, comprising or essentially consisting of endothelial cells, which first forms an inner part and has cavities, which preferably comprise hemogenic endothelium and/or SOX17-positive cells, a second layer directly adjacent the first layer and surrounding only a portion of the first layer, the second layer forming dense myocardium, optionally also endocardium, comprising or essentially consisting of NKX2.5-positive cardiomyocytes, and a third layer directly adjacent to the second layer only and surrounding only a portion of the second layer, which third layer forms epicardium and comprises or consists of epicardial cells and cardiomyocytes, has blood vessel-like structures and forms a loose tissue from epicardial cells and cardiomyocytes, wherein the third layer is covered by a directly adjacent fibroblast layer. Fig. 14D shows a cardiac organoid rotated around its vertical axis. The organoid is stained with DAPI and expresses eGFP under the control of the *NKX2.5* promoter. Fig. 14D demonstrates that cardiac organoids are radially symmetric, which means that they have a distinct front side (shown at 0°) and back side (shown at 180°; lateral view shown at 90°).

### Reference Example 3: Testing of the effect of a test compound on cardiac organoids

As an exemplary test compound, Thalidomide was used to test its influence on the development of cardiac organoids during the production process. Cardiac organoids were produced according to Example 1, and on day 1 (d1), i.e. after the incubation for 1 day in step i), 80 µg/mL Thalidomide was added to the culture medium. Micrographs of 7 cardiac organoids treated in parallel with Thalidomide (80 µg/mL Thalidomide) are shown in Fig. 15 along with micrographs of three cardiac organoids that were produced in parallel but without addition (Control) of the test compound.

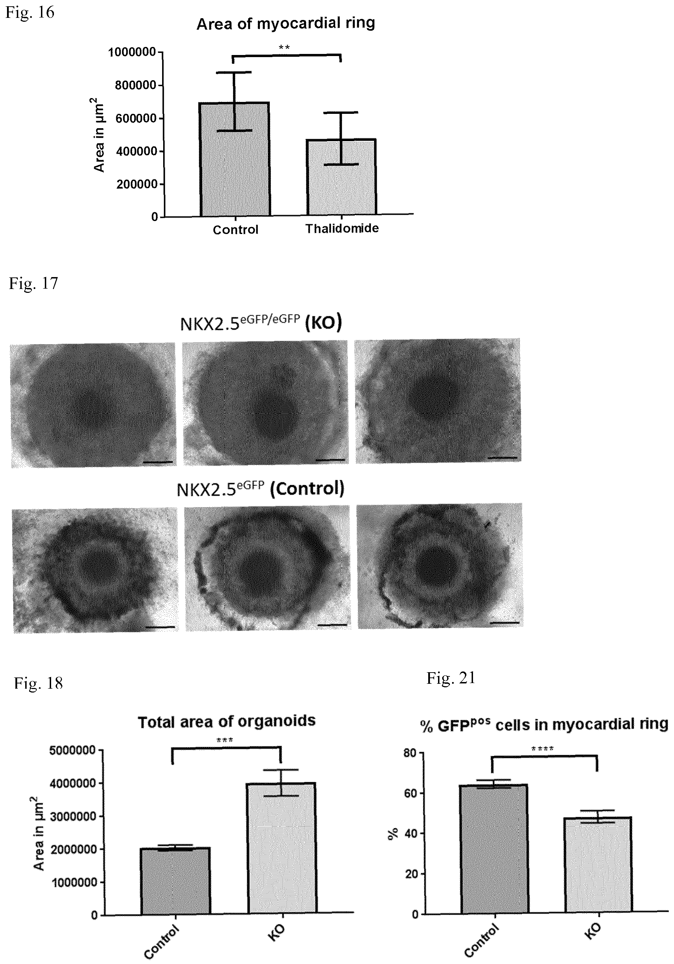

A comparison of the micrographs of the Thalidomide-treated cardiac organoids to the Control makes it clear that Thalidomide results in the absence of any NKX2.5-eGFP-positive cells on the outer layer, and that the myocardial ring of the Thalidomide-treated cardiac organoids is substantially thinner than in Controls. The average diameter of the myocardial ring in Controls was 276 µm, with Thalidomide it was 165 µm, which result is also shown in Fig. 16. This result on the exemplary test compound shows that the known effect of Thalidomide to cause also cardiac malformations during embryo development is also seen in the development of the cardiac organoids in the process according to the invention. This shows that the process for producing cardiac organoids is a suitable in vitro assay for the effect of test compounds, e.g. in respect of teratogenic effects.

### Reference Example 4: In vitro production of cardiac organoids from genetically aberrant PSC

As an example for PSC that carry a genetic aberrations, a human embryonic stem cell line HES3 NKX2.5^{eGFP/eGFP} was used, in which both NKX2.5 genes are dysfunctional (knock-out, NKX2.5-KO) and which expresses no functional NKX2.5 protein, but which expresses eGFP (enhanced green fluorescent protein) as a marker under the control of the NKX2.5 promoter. This cell line was produced as described by Anderson DJ et al. "NKX2-5 regulates human cardiomyogenesis via a HEY2 dependent transcriptional network", Nature Communications 2018,9: 1373 .

For comparison (Control), cardiac organoids were produced by the same process from a cell line that expressed normal levels of NKX2.5 protein and in addition contains the expression cassette encoding eGFP under the NKX2.5 promoter.

Cardiac organoids from the genetically aberrant, NKX2.5-KO PSC, and from the Control were produced by the process of the invention, as e.g. described in Example 1. Figures 17 to 21 show the analytical results of the NKX2.5-KO cardiac organoids in comparison to the Control cardiac organoids, both expressing eGFP under the control of the NKX2.5 promoter. Fig. 17 depicts fluorescence microscopic pictures of three exemplary cardiac organoids (scale bar 500 µm) for NKX2.5-KO and Control, showing that the cardiac organoids carrying the NKX2.5-KO are larger than the Control cardiac organoids. Total area occupied by the cardiac organoids was measured from the pictures quantitatively using ImageJ software. The measurement results are shown in Fig. 18, indicating significantly larger cardiac organoids for the NKX2.5-KO.

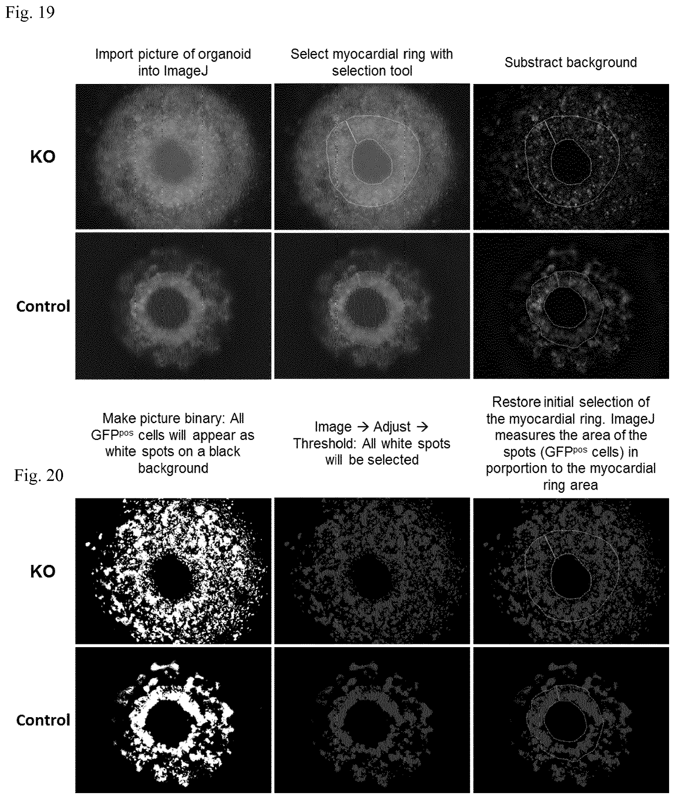

Figures 19 and 20 depict exemplary cardiac organoids in fluorescence microscopic pictures during optical analysis for determination of the compactness phenotype. As indicated in Fig. 19, the myocardial ring was optically isolated, and as indicated in Fig. 20, the area that was positive for eGFP in the ring area was determined. The area of the eGFP-positive ring area was divided by the total area determined for the myocardial ring, the result is shown in Fig. 21. This quantitative optical analysis was made using the ImageJ software (Wayne Rasband). The result depicted in Fig. 21 shows that the myocardial ring of Control cardiac organoids appears more compact and dense than the myocardial ring of the NKX2.5-KO cardiac organoids. The statistical analysis for the results of Fig. 21 was done using GraphPad Prism 7 software applying Student's t-test, cardiac organoids: Control n=5, NKX2.5-KO n=5.

These results, which are strongly reminiscent of both the muscle overgrowth and the lack of myocardial tissue compaction that was described for NKX2.5 defects in mouse and in human, show that cardiac organoids produced according to the invention from PSC that have a genetic aberration closely resemble the development of hearts observed in vivo and show structural aberrations resembling structural aberrations observed in vivo for the genetic aberration.

### Reference Example 5: In vitro production of cardiac organoids

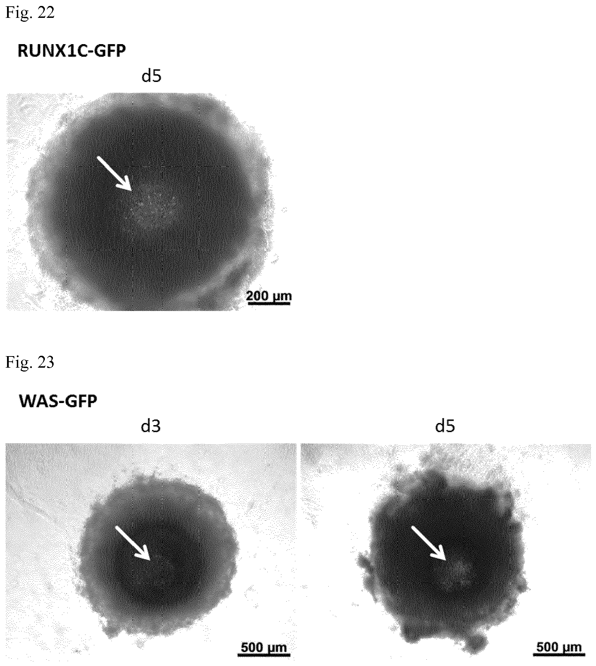

Cardiac organoids were produced by the process of the invention, using two different reporter cell lines, in which formation of hematopoietic cells derived from hemogenic endothelium is revealed by expression of GFP as a reporter, the expression of which is controlled by the hematopoietic cell-specific marker genes RUNX1C (Runt-related transcription factor 1c), cell line HES3 RUNX1C-GFP, or WAS (Wiskott-Aldrich Syndrome), iPS cell line WAS-GFP. The fluorescence microscopic of Fig. 22 depicts an exemplary cardiac organoid on day 5 after adding differentiation medium (d5) in step h), produced from the PSC expressing GFP under the control of the RUNX1C promoter.

The fluorescence microscopic picture of Fig. 23 depicts exemplary cardiac organoids on day 3 (d3) and day 5 (d5) after adding differentiation medium in step h), produced from the PSC expressing GFP under the control of the WAS promoter.

For both these cell lines, the expression of GFP in the inner part of the cardiac organoids shows that hemogenic endothelium is contained in the inner part of the cardiac organoids during the process of the invention. This indicates that the cardiac organoids produced by the process of the invention in their inner part contain hemogenic endothelium, which developed from the PSC originally used in the process. Hemogenic endothelium is known to be the origin of hematopoiesis.

### Reference Example 6: In vitro production of cardiac organoids and analysis for endothelial cells

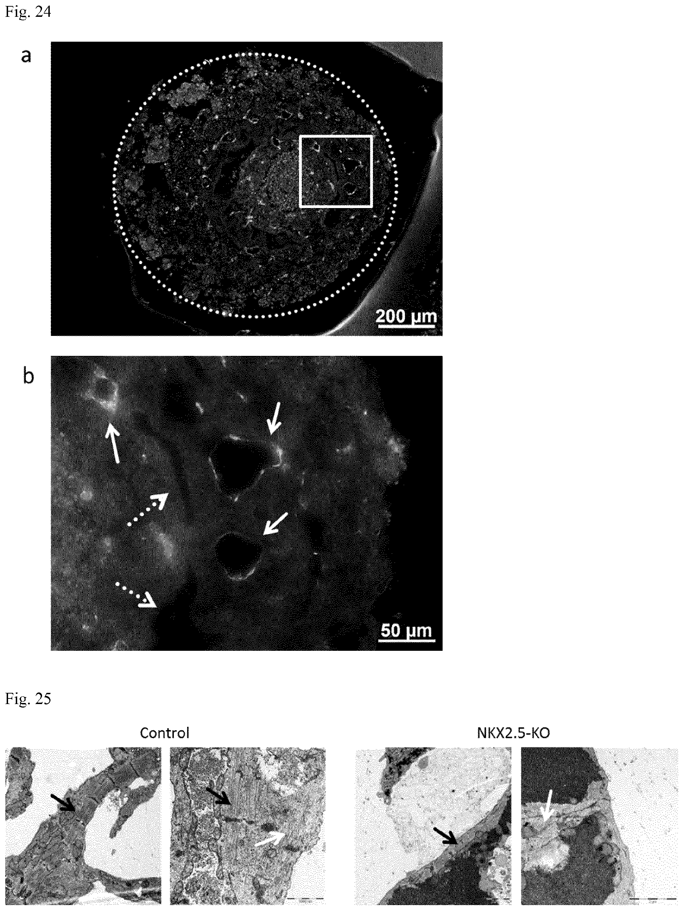

Cardiac organoids were produced as described in Example 1 and analysed for presence of endothelial cells. Paraffin-embedded cardiac organoids were stained with an endothelial cell-specific anti-CD31 antibody (CD31, Clone JC70A obtained from Agilent, Dako; secondary antibody: Cy3-AffiniPure Donkey Anti-Mouse IgG (H+L) obtained from Jackson ImmunoResearch Laboratories). Fig. 24 shows a fluorescence microscopic picture of this analysis, wherein the dotted line of Fig. 24a encloses a frontal section of a fixed exemplary cardiac organoid. Fig. 24b depicts an enlargement of the square highlighted in Fig. 24a. In this example, not all of the cavities are lined with CD31 positive cells. In Fig. 24b, solid arrows point at endothelial cell-lined cavities, whereas dotted arrows indicate other cavities without CD31 positive staining.

Positive staining of endothelial cells (represented by bright white stained cells) supports the finding that cardiac organoids of the invention contain endothelium-lined cavities resembling blood vessel-like structures.

Initial tests using immuno-staining of the endothelial marker vascular endothelial cadherin (VE-cadherin or CD144) by the labelled anti-CD 144 antibody human CD144 antibody BMS158 obtained from eBioscience were later found to not only detect blood-vessel like structures, but to also give false-positive signals.

Cardiac organoids were produced from NKX2.5-KO PSC and wild-type PSC (control) as described in Example 1. Fig. 25 (complete scale bar is 1000 nm) shows transmission electron microscopy pictures of sarcomeres in cardiomyocytes in NKX2.5-KO and control organoids. In accordance with the findings of Kasahara et al. 2001, loc. cit., for mouse embryos and neonatal mice, the cardiac organoids of the invention produced from NKX2.5-KO cells and control organoids produced from wild-type PSC contain well-organized (black arrows) as well as disorganized (white arrows) sarcomeres.

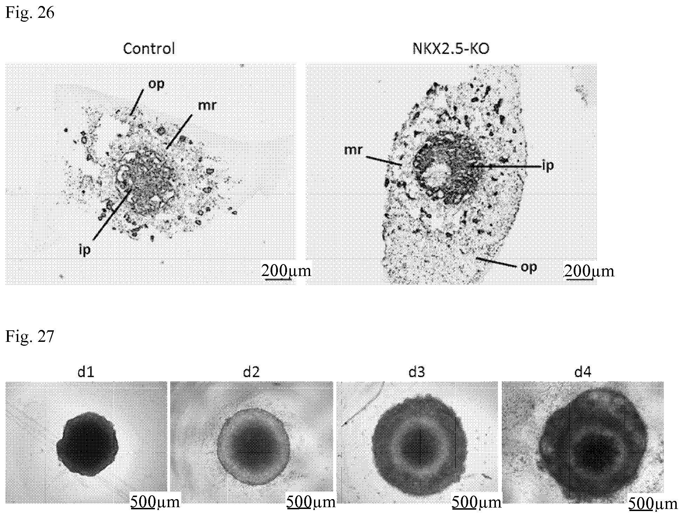

Fig. 26 shows paraffin sections of cardiac organoids produced from NKX2.5-KO and wild-type (control) PSC, stained with an anti-Ki67 antibody (Thermo Scientific). The anti-Ki67 antibody marks proliferating cells. Ki67 positive cells are shown as black dots, whereas negative cells appear light gray. The result demonstrates that in both, NKX2.5-KO and control organoids cells of the inner part (ip) and the outer part (op) are proliferative, whereas most of the cells of the myocardial ring (mr) are not proliferative (Ki67 negative). Notably, NKX2.5-KO organoids show a pronounced, highly proliferative outer part suggesting that the enlarged diameter of these organoids compared to controls (e.g. in relation to the quantitative assessment in Fig. 18 is, at least in part, caused by pronounced cell proliferation in the outer part, which is dominated by Calponin- and Vimentin-positive fibroblasts (see also Fig. 5 and Fig. 25), cardiomyocytes and epicardial cells (Fig. 6A, Fig. 7).

Further, cardiac organoids were produced according to the method of the invention from the HES3 MIXL1-GFP reporter cell line. In this cell line, previously described by Davis et al. Blood 2008, GFP is expressed under the control of the *MIXL1* gene promoter demarcating mesendodermal progenitor cells. These cardiac organoids of the invention demonstrate that the ring-like structure, which is typical for cardiac organoids generated with the HES3 NKX2.5-eGFP reporter cell line (see also Fig. 3), is readily reflected by the GFP pattern of MIXL1-GFP organoids at early stages of differentiation (day 1 to day 4). Microscopic analysis as shown in Fig. 27 (scale bar 500 µm) on day 1 (d1), day 2 (d2), day 3 (d3), and day 4 (d4) of differentiation suggests that the expression of MIXL1-GFP in cardiac organoids reaches its maximum on day 2 of differentiation and decreases thereafter. The ring-like structure of MIXL1-GFP positive cells covers the outer part of organoids on day 1 and day 2 of differentiation. On day 3 and day 4 of differentiation, the ring-like structure of MIXL1-GFP positive cells is restricted by MIXL-GFP-negative cells on the outside and in the inner core.

## Claims

1. Process for in vitro producing a cardiac organoid comprising
a) providing cultivated pluripotent stem cells (PSC) in a suspension in a first culture medium,
b) centrifuging the PSC in a first vessel having a U-shaped bottom to localize the PSC at the bottom of the first vessel,
c) incubating the PSC localized at the bottom of the first vessel under the first medium under cell culture conditions,
d) optionally removing the first medium from the PSC localized at the bottom of the first vessel,
e) embedding the PSC within hydrogel,
f) incubating the PSC embedded within the hydrogel for solidifying the hydrogel,
g) covering the cells embedded in the solidified hydrogel with a second cell culture medium and incubating under cell culture conditions,
h) removing the second medium from the cells and adding a third cell culture medium containing a first differentiation factor having activity to induce the WNT pathway and incubating under cell culture conditions for at least 6 h,
i) removing the third medium from the cells and adding a fourth cell culture medium not containing a differentiation factor or containing an agent neutralizing the activity of the first differentiation factor, and incubating under cell culture conditions for at least 6 h,
j) removing the medium from the cells and adding a fifth cell culture medium containing a second differentiation factor having activity to inhibit the WNT pathway and incubating under cell culture conditions for at least 1 d,
k) removing the fifth medium from the cells and adding a sixth medium not containing insulin and not containing a differentiation factor having activity to induce or to inhibit the WNT pathway, and incubating under cell culture conditions for at least 1 d,
l) removing the sixth medium from the cells and adding a seventh cell culture medium containing insulin and incubating under cell culture conditions for at least 1 d,
m) and subsequently change the seventh medium for fresh cell culture medium at least every 2 d.

2. Process according to claim 1, **characterized in that** the incubation in steps a) to m) is under essentially static conditions.

3. Process according to one of the preceding claims, **characterized in that** in step e) hydrogel is positioned in a second vessel and the aggregated PSC are transferred from the first vessel into the hydrogel for embedding the aggregate of PSC within the hydrogel.

4. Process according to one of the preceding claims, **characterized in that** in step g) the PSC embedded in the solidified hydrogel are incubated with a second cell culture medium for at approximately 2 d.

5. Process according to one of the preceding claims, **characterized in that** in step h) the first differentiation factor is CHIR99021.

6. Process according to one of the preceding claims, **characterized in that** in step j) the second differentiation factor is IWP-2.

7. Process according to one of the preceding claims, **characterized in that** in at least one of step h) the third medium contains B27 supplement without insulin, of step i) the fourth medium contains B27 supplement without insulin, of step j) the fifth medium contains B27 supplement without insulin, of step k) the sixth medium contains B27 supplement without insulin, and of step 1) the seventh medium contains B27 supplement containing insulin.

8. Process according to one of the preceding claims, **characterized by** dissolving the hydrogel after one of the steps 1) or m).

9. Process according to one of the preceding claims, **characterized by** adding at least one test compound to at least one medium of the process and analysing the cardiac organoid in comparison to a cardiac organoid produced in a parallel process performed without the addition of the test compound.

10. Process according to claim 9, wherein the structure and/or localization of cell types of the cardiac organoids is analysed.

11. Process according to one of the preceding claims, **characterized in that** the PSC have a genetic aberration.

12. Cardiac organoid obtainable by a process according to one of the preceding claims, **characterized in that** the organoid has a first layer that forms an inner part and has cavities, which first layer is at least in part surrounded by a second layer comprising endothelial cells and cardiomyocytes, which second layer is at least in part surrounded by a third layer comprising cardiomyocytes and epicardial cells, which third layer is at least in part surrounded by a fourth layer comprising fibroblast cells.

13. Cardiac organoid according to claim 12, **characterized in that** the first layer completely surrounds the cavities and is circumferentially closed, that the second layer surrounds the first layer only partially, and that the third layer only surrounds the second layer and that the fourth layer only surrounds the third layer.

14. Cardiac organoid according to one of claims 12 to 13, **characterized in that** the cavities of the inner part contain foregut endoderm, blood vessels and hemogenic endothelium.

15. Cardiac organoid according to one of claims 12 to 14, **characterized in that** it generally has a sphere shape, in which the outer surface is formed by a portion of the first layer, a portion of the second layer and the entire third and/or fourth layer.

16. Cardiac organoid according to one of claims 12 to 15, **characterized in that** it has an outer diameter in the range from 0.5 to 5 mm.

17. Cardiac organoid according to one of claims 12 to 16 for use as an implant in the treatment of a patient.

## Patentansprüche

1. Verfahren zur in vitro-Herstellung eines Herzorganoids, umfassend
a) Bereitstellung von kultivierten pluripotenten Stammzellen (PSC) in einer Suspension in einem ersten Kulturmedium,
b) Zentrifugieren der PSC in einem ersten Behälter mit einem U-förmigen Boden, um die PSC am Boden des ersten Behälters zu lokalisieren,
c) Inkubation der am Boden des ersten Gefäßes befindlichen PSC unter dem ersten Medium unter Zellkulturbedingungen,
d) optionales Entfernen des ersten Mediums von den PSC, die sich am Boden des ersten Gefäßes befinden,
e) Einbettung der PSC in Hydrogel,
f) Inkubation der in das Hydrogel eingebetteten PSC zur Verfestigung des Hydrogels,
g) Bedecken der in das verfestigte Hydrogel eingebetteten Zellen mit einem zweiten Zellkulturmedium und Inkubieren unter Zellkulturbedingungen,
h) Entfernen des zweiten Mediums von den Zellen und Zugabe eines dritten Zellkulturmediums, das einen ersten Differenzierungsfaktor mit einer Aktivität zur Induktion des WNT-Signalwegs enthält, und Inkubation unter Zellkulturbedingungen für mindestens 6 h,
i) Entfernen des dritten Mediums von den Zellen und Zugabe eines vierten Zellkulturmediums, das keinen Differenzierungsfaktor oder ein Mittel enthält, das die Aktivität des ersten Differenzierungsfaktors neutralisiert, und Inkubation unter Zellkulturbedingungen für mindestens 6 h,
j) Entfernen des Mediums von den Zellen und Zugabe eines fünften Zellkulturmediums, das einen zweiten Differenzierungsfaktor mit Aktivität zur Hemmung des WNT-Signalwegs enthält, und Inkubation unter Zellkulturbedingungen für mindestens 1 d,
k) Entfernen des fünften Mediums von den Zellen und Hinzufügen eines sechsten Mediums, das kein Insulin und keinen Differenzierungsfaktor mit einer Aktivität zur Induktion oder Hemmung des WNT-Signalwegs enthält, und Inkubieren unter Zellkulturbedingungen für mindestens 1 d,
l) Entfernen des sechsten Mediums von den Zellen und Zugabe eines siebten insulinhaltigen Zellkulturmediums und Inkubation unter Zellkulturbedingungen für mindestens 1 d,
m) und anschließend mindestens alle 2 Tage Austauschen des siebten Mediums gegen frisches Zellkulturmedium.

2. Verfahren nach Anspruch 1, **dadurch gekennzeichnet, dass** die Inkubation in den Schritten a) bis m) unter im Wesentlichen statischen Bedingungen erfolgt.

3. Verfahren nach einem der vorhergehenden Ansprüche, **dadurch gekennzeichnet, dass** in Schritt e) Hydrogel in einem zweiten Gefäß positioniert wird und die aggregierten PSC aus dem ersten Gefäß in das Hydrogel überführt werden, um das Aggregat der PSC in das Hydrogel einzubetten.

4. Verfahren nach einem der vorhergehenden Ansprüche, **dadurch gekennzeichnet, dass** in Schritt g) die in das verfestigte Hydrogel eingebetteten PSC für etwa 2 d mit einem zweiten Zellkulturmedium inkubiert werden.

5. Verfahren nach einem der vorhergehenden Ansprüche, **dadurch gekennzeichnet, dass** in Schritt h) der erste Differenzierungsfaktor CHIR99021 ist.

6. Verfahren nach einem der vorhergehenden Ansprüche, **dadurch gekennzeichnet, dass** in Schritt j) der zweite Differenzierungsfaktor IWP-2 ist.

7. Verfahren nach einem der vorhergehenden Ansprüche, **dadurch gekennzeichnet, dass** in mindestens einem von Schritt h) das dritte Medium einen B27-Zusatz ohne Insulin enthält, von Schritt i) das vierte Medium einen B27-Zusatz ohne Insulin enthält, von Schritt j) das fünfte Medium einen B27-Zusatz ohne Insulin enthält, von Schritt k) das sechste Medium einen B27-Zusatz ohne Insulin enthält und von Schritt l) das siebte Medium einen B27-Zusatz mit Insulin enthält.

8. Verfahren nach einem der vorhergehenden Ansprüche, **gekennzeichnet durch** Auflösen des Hydrogels nach einem der Schritte l) oder m).

9. Verfahren nach einem der vorhergehenden Ansprüche, **gekennzeichnet durch** Zugeben mindestens einer Testverbindung zu mindestens einem Medium des Verfahrens und Analysieren des Herzorganoids im Vergleich zu einem Herzorganoid, das in einem parallel durchgeführten Verfahren ohne die Zugabe der Testverbindung hergestellt wurde.

10. Verfahren nach Anspruch 9, wobei die Struktur und/oder die Lokalisierung von Zelltypen der Herzorganoide analysiert wird.

11. Verfahren nach einem der vorhergehenden Ansprüche, **dadurch gekennzeichnet, dass** die PSC eine genetische Aberration aufweisen.

12. Herzorganoid, erhältlich durch ein Verfahren nach einem der vorhergehenden Ansprüche, **dadurch gekennzeichnet, dass** das Organoid eine erste, einen inneren Teil bildende und Hohlräume aufweisende Schicht aufweist, die zumindest teilweise von einer zweiten, Endothelzellen und Kardiomyozyten umfassenden Schicht umgeben ist, die zumindest teilweise von einer dritten, Kardiomyozyten und Epikardzellen umfassenden Schicht umgeben ist, die zumindest teilweise von einer vierten, Fibroblastenzellen umfassenden Schicht umgeben ist.

13. Herzorganoid nach Anspruch 12, **dadurch gekennzeichnet, dass** die erste Schicht die Hohlräume vollständig umgibt und umfänglich geschlossen ist, dass die zweite Schicht die erste Schicht nur teilweise umgibt, und dass die dritte Schicht nur die zweite Schicht umgibt und dass die vierte Schicht nur die dritte Schicht umgibt.

14. Herzorganoid nach einem der Ansprüche 12 bis 13, **dadurch gekennzeichnet, dass** die Hohlräume des inneren Teils Vormagenendoderm, Blutgefäße und hämogenes Endothel enthalten.

15. Herzorganoid nach einem der Ansprüche 12 bis 14, **dadurch gekennzeichnet, dass** es im Allgemeinen eine Kugelform aufweist, bei der die äußere Oberfläche von einem Teil der ersten Schicht, einem Teil der zweiten Schicht und der gesamten dritten und/oder vierten Schicht gebildet wird.

16. Herzorganoid nach einem der Ansprüche 12 bis 15, **dadurch gekennzeichnet, dass** es einen Außendurchmesser im Bereich von 0,5 bis 5 mm aufweist.

17. Herzorganoid nach einem der Ansprüche 12 bis 16 zur Verwendung als Implantat bei der Behandlung eines Patienten.

## Revendications

1. Procédé de production in vitro d'un organoïde cardiaque comprenant
a) fournir des cellules souches pluripotentes cultivées (PSC) en suspension dans un premier milieu de culture,
b) centrifuger le PSC dans un premier récipient ayant un fond en forme de U pour localiser le PSC au fond du premier récipient,
c) incubation du PSC localisé au fond du premier récipient sous le premier milieu dans des conditions de culture cellulaire,
d) en retirant éventuellement le premier milieu du PSC localisé au fond du premier récipient,
e) l'intégration du PSC dans l'hydrogel,
f) incubation du PSC noyé dans l'hydrogel pour solidifier l'hydrogel,
g) recouvrir les cellules noyées dans l'hydrogel solidifié avec un second milieu de culture cellulaire et les incuber dans des conditions de culture cellulaire,
h) retirer le second milieu des cellules et ajouter un troisième milieu de culture cellulaire contenant un premier facteur de différenciation ayant une activité d'induction de la voie WNT et incuber dans des conditions de culture cellulaire pendant au moins 6 heures,
i) retirer le troisième milieu des cellules et ajouter un quatrième milieu de culture cellulaire ne contenant pas de facteur de différenciation ou contenant un agent neutralisant l'activité du premier facteur de différenciation, et incuber dans des conditions de culture cellulaire pendant au moins 6 heures,
j) retirer le milieu des cellules et ajouter un cinquième milieu de culture cellulaire contenant un second facteur de différenciation ayant une activité d'inhibition de la voie WNT et incuber dans des conditions de culture cellulaire pendant au moins 1 jour,
k) retirer le cinquième milieu des cellules et ajouter un sixième milieu ne contenant pas d'insuline et ne contenant pas de facteur de différenciation ayant une activité pour induire ou inhiber la voie WNT, et incuber dans des conditions de culture cellulaire pendant au moins 1 jour,
l) retirer le sixième milieu des cellules et ajouter un septième milieu de culture cellulaire contenant de l'insuline et incuber dans des conditions de culture cellulaire pendant au moins 1 jour,
m) et changer ensuite le septième milieu pour un milieu de culture cellulaire frais au moins tous les 2 jours.

2. Procédé selon la revendication 1, **caractérisé en ce que** l'incubation dans les étapes a) à m) est dans des conditions essentiellement statiques.

3. Procédé selon l'une des revendications précédentes, **caractérisé en ce que**, à l'étape e), l'hydrogel est positionné dans un deuxième récipient et les PSC agrégés sont transférés du premier récipient dans l'hydrogel pour noyer l'agrégat de PSC dans l'hydrogel.

4. Procédé selon l'une des revendications précédentes, **caractérisé en ce que** dans l'étape g), les PSC noyés dans l'hydrogel solidifié sont incubés avec un second milieu de culture cellulaire pendant approximatirement 2 jours.

5. Procédé selon l'une des revendications précédentes, **caractérisé en ce que** dans l'étape h) le premier facteur de différenciation est CHIR99021.

6. Procédé selon l'une des revendications précédentes, **caractérisé en ce que** dans l'étape j) le deuxième facteur de différenciation est IWP-2.

7. Procédé selon l'une des revendications précédentes, **caractérisé en ce que** dans au moins l'une de l'étape h) le troisième milieu contient du supplément B27 sans insuline, de l'étape i) le quatrième milieu contient du supplément B27 sans insuline, de l'étape j) le cinquième milieu contient du supplément B27 sans insuline, de l'étape k) le sixième milieu contient du supplément B27 sans insuline, et de l'étape l) le septième milieu contient du supplément B27 contenant de l'insuline.

8. Procédé selon l'une des revendications précédentes, **caractérisé par** la dissolution de l'hydrogel après l'une des étapes l) ou m).

9. Procédé selon l'une des revendications précédentes, **caractérisé par** l'ajout d'au moins un composé test à au moins un milieu du procédé et l'analyse de l'organoïde cardiaque en comparaison avec un organoïde cardiaque produit dans un procédé parallèle réalisé sans l'ajout du composé test.

10. Procédé selon la revendication 9, dans lequel on analyse la structure et/ou la localisation des types de cellules des organoïdes cardiaques.

11. Procédé selon l'une des revendications précédentes, **caractérisé en ce que** les PSC présentent une aberration génétique.

12. Organoïde cardiaque pouvant être obtenu par un procédé selon l'une des revendications précédentes, **caractérisé en ce que** l'organoïde présente une première couche qui forme une partie interne et présente des cavités, laquelle première couche est au moins en partie entourée d'une deuxième couche comprenant des cellules endothéliales et des cardiomyocytes, laquelle deuxième couche est au moins en partie entourée d'une troisième couche comprenant des cardiomyocytes et des cellules épicardiques, laquelle troisième couche est au moins en partie entourée d'une quatrième couche comprenant des cellules de fibroblastes.

13. Organoïde cardiaque selon la revendication 12, **caractérisé en ce que** la première couche entoure complètement les cavités et est fermée circonférentiellement, que la deuxième couche n'entoure que partiellement la première couche, et que la troisième couche n'entoure que la deuxième couche et que la quatrième couche n'entoure que la troisième couche.

14. Organoïde cardiaque selon l'une des revendications 12 à 13, **caractérisé en ce que** les cavités de la partie interne contiennent de l'endoderme de foregut, des vaisseaux sanguins et de l'endothélium hémogène.

15. Organoïde cardiaque selon l'une des revendications 12 à 14, **caractérisé en ce qu'**il présente généralement une forme de sphère, dans laquelle la surface extérieure est formée par une partie de la première couche, une partie de la deuxième couche et la totalité de la troisième et/ou de la quatrième couche.

16. Organoïde cardiaque selon l'une des revendications 12 à 15, **caractérisé en ce qu'**il présente un diamètre extérieur compris entre 0,5 et 5 mm.

17. Organoïde cardiaque selon l'une des revendications 12 à 16 destiné à être utilisé comme implant dans le traitement d'un patient.
